# EUROPEAN PATENT SPECIFICATION

(11) **EP 4 349 267 B1**
(45) Date of publication and mention of the grant of the patent: **28.10.2026**
(21) Application number: 22815616.2
(22) Date of filing: 09.03.2022
(51) Int. Cl.: G06T 7/11, G06T 7/162, A61B 8/14

(54) **COMPUTER PROGRAM, LEARNING MODEL GENERATION METHOD, IMAGE PROCESSING METHOD, AND IMAGE PROCESSING DEVICE**
COMPUTERPROGRAMM, LERNMODELLERZEUGUNGSVERFAHREN, BILDVERARBEITUNGSVERFAHREN UND BILDVERARBEITUNGSVORRICHTUNG
PROGRAMME INFORMATIQUE, PROCÉDÉ DE GÉNÉRATION DE MODÈLE D'APPRENTISSAGE, PROCÉDÉ DE TRAITEMENT D'IMAGE ET DISPOSITIF DE TRAITEMENT D'IMAGE

(30) Priority: 03.06.2021 JP 2021093624
(43) Date of publication of application: 10.04.2024
(73) Proprietor: Furuno Electric Co., Ltd., Nishinomiya-City, Hyogo 662-8580 (JP)
(72) Inventor: ISERI, Kensuke, Nishinomiya-City, Hyogo 6628580 (JP)
(74) Representative: Müller Hoffmann & Partner
(86) International application number: PCT/JP2022/010258
(87) International publication number: WO 2022/254859

(56) References cited:
- WO-A1-2019/239293
- WO-A1-2020/012884
- JP-A- 2017 029 343
- US-A1- 2018 276 815
- US-A1- 2020 320 713
- TITINUNT KITRUNGROTSAKUL ET AL: "Interactive Deep Refinement Network for Medical Image Segmentation", ARXIV.ORG, CORNELL UNIVERSITY LIBRARY, 201 OLIN LIBRARY CORNELL UNIVERSITY ITHACA, NY 14853, 27 June 2020 (2020-06-27), XP081707881

## Description

The present invention relates to a computer program, a learning model generation method, an image processing method, and an image processing device.

In order to assist doctors in diagnosis, a learning model generated using machine learning is used to automatically extract a desired region in a medical image. In order to generate such a learning model, teacher data has to be created in advance.

Patent Literature 1 discloses a teacher data generation device that generates teacher data in which a correct label is attached to each pixel of each of multiple images.

### Patent Literature

[Patent Literature 1] Japanese Patent Application Laid-Open No. 2019-101535. US 2020/320713 A1 is essentially concerned with a binary segmentation of foreground versus background in medical images. US 2018/ 276815 A1 describes an interactive segmentation with user correction. The paper by Titinunt Kitrungrotsakul et al. having the title: "Interactive Deep Refinement Network for Medical Image Segmentation". ARXIV.ORG, Cornell University Ithaca, NY 14853, as published on 27 June 2020, introduces a segmentation refinement and assumes a single target region.

However, in order to generate a learning model which is capable of identifying regions (areas) with high accuracy, a large amount of data for learning, including teacher data, is required. Therefore, if sufficient data for learning cannot be prepared, there is a possibility that the accuracy of identifying the region decreases. In addition, when a clear medical image cannot be acquired, the accuracy of identifying the region also decreases.

In view of such circumstances, the present invention is intended to provide a computer program, a learning model generation method, an image processing method, and an image processing device that are capable of identifying a region with high accuracy.

### Solution to Problem

Problems with the prior art are solved by the subject-matter of the independent claims.

A computer program according to the present invention comprises acquiring a medical image including a plurality of regions; identifying the candidate area for each of the regions by inputting the medical image to a learning model for identifying a candidate area for each of the regions when the medical image is input; and identifying an area for each of the regions based on seed pixels in the candidate area for each of the regions.

The computer program according to the present invention comprises displaying the area for each of the regions which is identified in a different manner.

The computer program according to the present invention comprises identifying the area for each of the regions by a GrowCut method, a Graph-cuts method, a Grab-cut method, or a Random Walker method based on the seed pixels in the candidate area for each of the regions which is identified.

The computer program according to the present invention comprises selecting the seed pixels based on a degree of accuracy for pixels in the candidate area for each of the regions which is identified to be in each of the regions.

The computer program according to the present invention comprises selecting the seed pixels from a largest candidate area among the plurality of candidate areas when there are a plurality of candidate areas for each of the regions that are identified.

The computer program according to the present invention comprises retraining the learning model based on the medical image including the plurality of regions and area data indicating the area for each of the regions which is identified.

The computer program according to the present invention comprises accepting a correction to the area for each of the regions which is identified from a user.

The computer program according to the present invention comprises retraining the learning model based on the medical image which is acquired and area data indicating the area for each of the regions for which the correction is accepted.

In the computer program according to the present invention, the medical image includes an ultrasound image.

In the computer program according to the present invention, an imaging target of the medical image is any of a thigh, an upper arm, and an abdomen.

A learning model generation method according to the present invention includes: acquiring first training data including a medical image including a plurality of regions and a candidate area for each of the regions; generating a learning model to identify the candidate area for each of the regions when the medical image including the plurality of regions is input, based on the first training data; identifying an area for each of the regions based on seed pixels in the candidate area for each of the regions which is identified by the learning model; and generating the learning model to identify the area for each of the regions when the medical image including the plurality of regions is input, based on second training data including the medical image including the plurality of regions and area data indicating the area for each of the regions which is identified.

A learning model generation method according to the present invention includes: acquiring data including a medical image including a plurality of regions and a candidate area for each of the regions; identifying an area for each of the regions based on seed pixels in the candidate area; and generating a learning model to identify the area for each of the regions when the medical image including the plurality of regions is input, based on training data including the medical image including the plurality of regions and area data indicating the area for each of the regions which is identified.

An image processing method according to the present invention includes: acquiring a medical image including a plurality of regions; identifying the candidate area for each of the regions by inputting the medical image to a learning model for identifying a candidate area for each of the regions when the medical image is input; and identifying an area for each of the regions based on seed pixels in the candidate area for each of the regions.

An image processing device according to the present invention includes: an acquisition unit configured to acquire a medical image including a plurality of regions; an area identification unit configured to identify a candidate area for each of the regions by inputting the medical image to a learning model for identifying a candidate area for each of the regions when the medical image is input; and a region identification unit configured to identify an area for each of the regions based on seed pixels in the candidate area for each of the regions which is identified.

The present invention enables identification of a region with high accuracy.
FIG. 1 is a diagram showing an example of the configuration of an image processing device of the embodiment.
FIG. 2 is a diagram showing an example of the learning model.
FIG. 3 is a diagram showing an example of processing performed by the area identification unit.
FIG. 4 is a diagram showing a first example of a seed pixel selection method.
FIG. 5 is a diagram showing a second example of a seed pixel selection method.
FIG. 6 is a diagram showing an example of processing performed by the region identification unit.
FIG. 7 is a diagram showing an example of the GrowCut method.
FIG. 8 is a diagram showing an example of a region identification screen.
FIG. 9 is a diagram showing an example of an area correction screen of a region.
FIG. 10 is a diagram showing another example of the configuration of an image processing device of the embodiment.
FIG. 11 is a flowchart showing a first example of the processing procedure of the image processing device.
FIG. 12 is a flowchart showing a second example of the processing procedure of the image processing device.
FIG. 13 is a flowchart showing a first example of the processing procedure of the learning model generation method.
FIG. 14 is a flowchart showing a second example of the processing procedure of the learning model generation method.

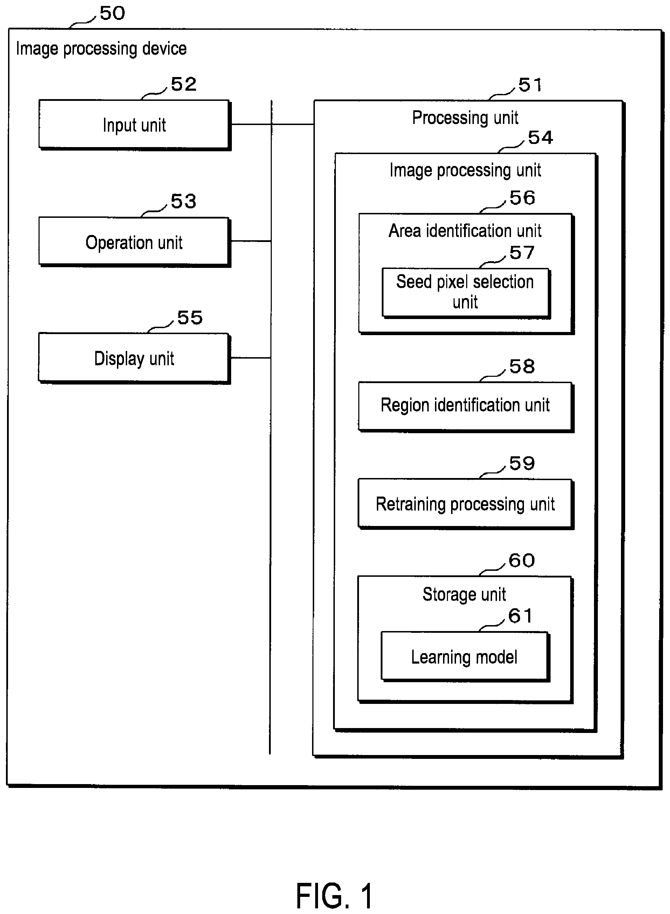

An embodiment of the present invention is to be described below. FIG. 1 is a diagram showing an example of the configuration of an image processing device 50 of the embodiment. The image processing device 50 includes a processing unit 51 that controls the entire device, an input unit 52, an operation unit 53, and a display unit 55. The processing unit 51 includes an image processing unit 54. The image processing unit 54 includes an area identification unit 56, a region identification unit 58, a retraining processing unit 59, and a storage unit 60. The area identification unit 56 includes a seed pixel selection unit 57. The storage unit 60 stores a learning model 61 (which is also referred to as a trained model).

The processing unit 51 may be configured by a Central Processing Unit (CPU), a Read Only Memory (ROM), a Random Access Memory (RAM), and the like.

The input unit 52 functions as an acquisition unit that acquires medical image data (medical images) via wired communication or wireless communication. Medical images include, for example, ultrasound images, MRI images, CT images, Positron Emission Tomography (PET) images, microscope images, and the like. The input unit 52 is capable of acquiring, for example, medical images from a device such as an ultrasonic diagnostic device, a computed tomography device, a magnetic resonance diagnostic device, an X-ray imaging device, an angiography X-ray diagnostic device, a PET examination device, or an electron microscope. The input unit 52 stores the acquired medical image data in the storage unit 60 and outputs the acquired medical image data to the area identification unit 56.

The operation unit 53 is composed of, for example, a hardware keyboard, a mouse, etc., and is capable of operating icons displayed on the display unit 55, moving and operating a cursor, and inputting characters. It should be noted that the operation unit 53 may be configured by a touch panel.

The display unit 55 may be configured by a liquid crystal panel, an organic Electro Luminescence (EL) display, or the like.

The area identification unit 56 reads the learning model 61 stored in the storage unit 60 and inputs the medical image data acquired by the input unit 52 to the read learning model 61. When the medical image data is input, the learning model 61 identifies a candidate area for each of multiple regions included in the medical image, and outputs the identified candidate area image data. The candidate area image data includes a code (a region label) indicating to which region each pixel of the medical image belongs, and the degree of accuracy (reliability) that the pixel is the code.

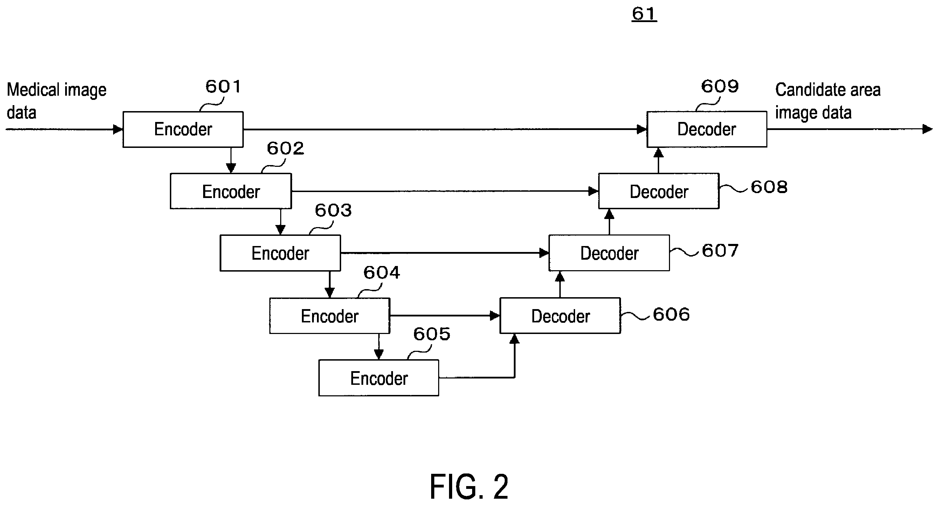

FIG. 2 is a diagram showing an example of the learning model 61. The learning model 61 may be configured by U-Net, for example. The learning model 61 includes encoders 601 to 605 and decoders 606 to 609. The learning model 61 repeats convolution processing with the encoders 601 to 605 on the input medical image data. Upsampling (deconvolution) processing of the image convoluted by the encoder 605 is repeated with the decoders 606 to 609. When decoding the convolved image, processing is performed to add the feature maps generated by the encoders 604 to 601 to the image to be deconvolved. The learning model 61 outputs a segmentation image (a candidate area image) of multiple regions included in the input medical image. From the segmentation image, it is possible to obtain the boundaries of each of the regions and the boundaries of the whole region including the regions.

The above configuration enables retention of the position information which is lost due to the convolution processing, and output of a more accurate segmentation (which pixel is which region). The learning model 61 is not limited to U-Net and may be, for example, Generative Adversarial Network (GAN), SegNet, etc.

The learning model 61 may be generated as follows. Training data (first training data) includes medical image data including multiple regions and teacher data. Segmentation image data created by making annotation based on the medical image data is used as the teacher data. The segmentation image is an image that indicates to which region each pixel of the medical image belongs. A large amount of the medical image data and the teacher data is prepared to machine-learn the learning model 61. That is, when the medical image data is input to the learning model 61, the learning model 61 may be generated by updating the internal parameters of the learning model 61 so that the candidate area image data output by the learning model 61 approaches the teacher data. The learning model 61 may be generated by an external learning device, and the generated learning model 61 may be stored in the storage unit 60. A learning processing unit may be provided in the image processing device 50, and the learning model 61 may be generated by the image processing device 50.

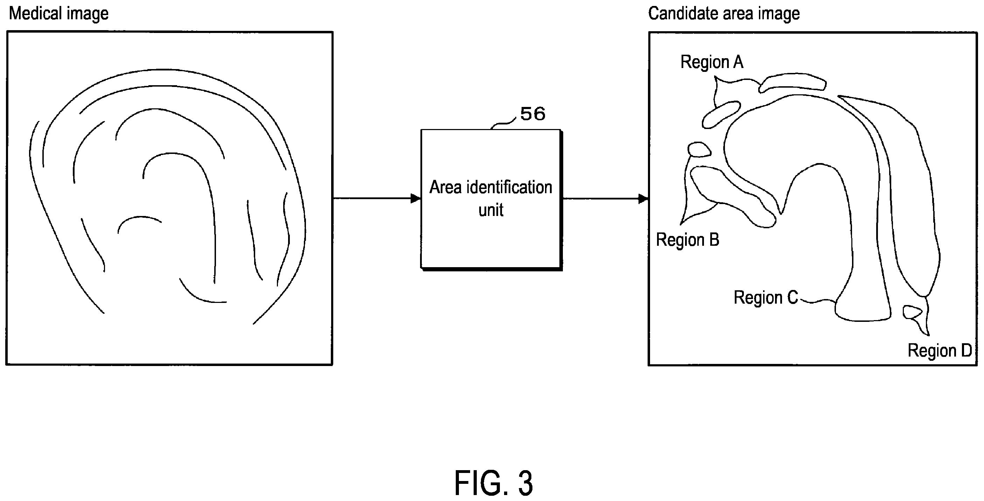

FIG. 3 is a diagram showing an example of processing performed by the area identification unit 56. In the following, the quadriceps femoris muscle is to be described as an example of the whole region. As shown in FIG. 3, when a medical image in which the quadriceps femoris muscle is captured is input to the area identification unit 56, the area identification unit 56 outputs a candidate area image. The candidate area image data includes candidate area data for each of region A, region B, region C, and region D as multiple regions. For the quadriceps femoris muscle, the regions A to D are rectus femoris muscle, vastus medialis muscle, vastus intermedius muscle, and vastus lateralis muscle, respectively.

If the medical image input to the area identification unit 56 is partially unclear, such as an ultrasound image, or if the image is not clear as a whole, the candidate area image output by the area identification unit 56 is not an area in which the same regions are continuous such as the regions A, B, and D, but multiple separated areas. Also, a gap is generated between each of the regions A to D, and the boundaries of each of the regions become unclear. In this way, missing areas and outlying areas occur in the candidate area image. Also, when the amount of training data used for learning by the learning model 61 is not sufficient, a candidate area image such as the candidate area image shown in FIG. 3 is output.

In the embodiment, by using the region identification unit 58 in addition to the area identification unit 56 (the learning model 61), multiple regions in the medical image may be identified with a high degree of accuracy. This point is to be described below.

The seed pixel selection unit 57 selects seed pixels (seed points) based on the candidate area image output by the area identification unit 56. The seed pixel selection unit 57 outputs the selected seed pixels to the region identification unit 58. It should be noted that seed pixels may be manually selected from among the candidate area image output by the area identification unit 56.

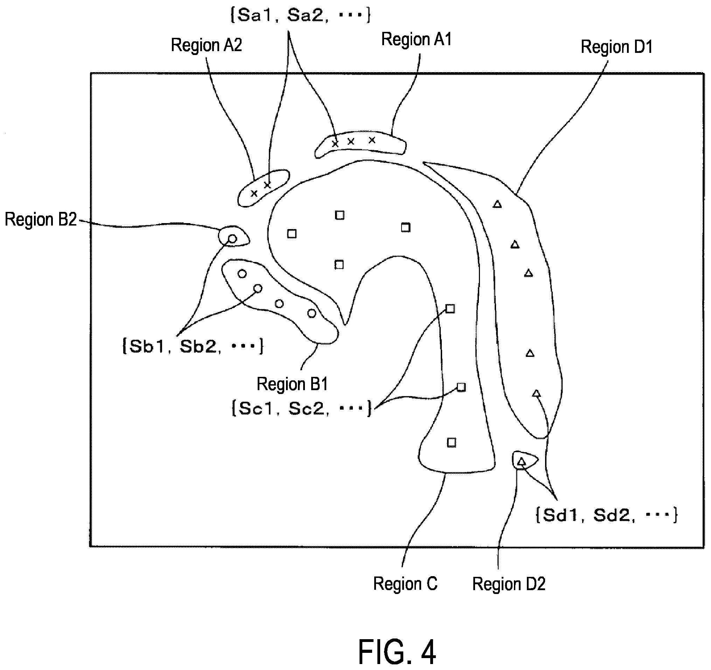

FIG. 4 is a diagram showing a first example of a seed pixel selection method. The candidate area image data includes a code (a region label) indicating to which region each pixel of the medical image belongs, and the degree of accuracy (reliability) that the pixel is the code. Therefore, pixels of which the degree of accuracy is equal to or higher than a particular threshold may be selected as seed pixels for each of the regions. In the example of FIG. 4, seed pixels {Sa1, Sa2, ...} are selected for which the degree of accuracy of the pixels in regions A1 and A2 separated from the region A is greater than or equal to the particular threshold. Seed pixels {Sb1, Sb2, ...} are selected for which the degree of accuracy of the pixels in regions B1 and B2 separated from the region B is greater than or equal to the particular threshold. Seed pixels {Sc1, Sc2, ...} are selected for which the degree of accuracy of the pixels in the region C is greater than or equal to the particular threshold. Seed pixels {Sd1, Sd2, ...} are selected for which the degree of accuracy of the pixels in regions D1 and D2 separated from the region D is greater than or equal to the particular threshold. In this way, the seed pixel selection unit 57 may select seed pixels based on the degree of accuracy that the pixels in the identified candidate area are each of the regions.

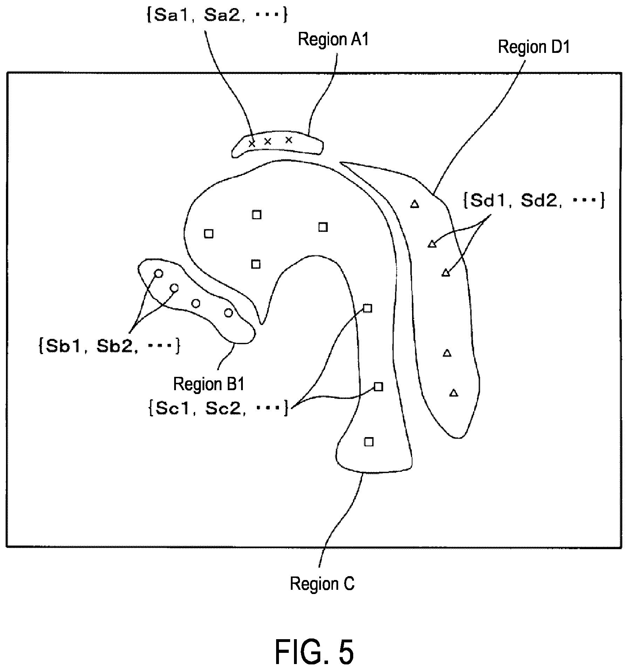

FIG. 5 is a diagram showing a second example of a seed pixel selection method. When there are multiple candidate areas for each of the regions identified, the seed pixel selection unit 57 may select the seed pixels from the largest candidate area among the candidate areas. In the example of FIG. 5, the region A1 and the region A2 are identified as candidate areas for the region A. Among the regions A1 and A2, the seed pixels {Sa1, Sa2, ...} are selected for which the degree of accuracy of the pixels of the region A1, which is the largest candidate area, is equal to or higher than the particular threshold. The region B1 and the region B2 are identified as candidate areas for the region B. Among the regions B1 and B2, the seed pixels {Sb1, Sb2, ...} are selected for which the degree of accuracy of pixels in the region B1, which is the largest candidate area, is equal to or higher than the particular threshold. The region D1 and the region D2 are identified as candidate areas for the region D. Among the regions D1 and D2, the seed pixels {Sd1, Sd2, ...} are selected for which the degree of accuracy of the pixels of the region D1, which is the largest candidate area, is equal to or higher than the particular threshold. The number of multiple candidate areas of the same region is not limited to two as in FIG. 5. Also, the number of seed pixels is not limited to the numbers illustrated in FIGS. 4 and 5. Although the second example shown in FIG. 5 is not critical, suitable seed pixels may be obtained when there are multiple candidate areas of the same region that are separated from each other. The seed pixel selection unit 57 outputs the seed point image to the region identification unit 58.

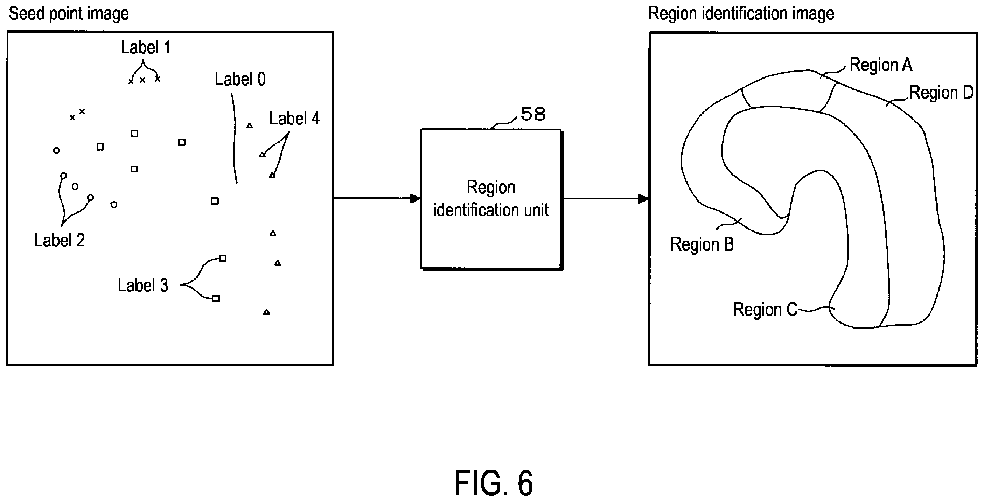

FIG. 6 is a diagram showing an example of processing performed by the region identification unit 58. As shown in FIG. 6, when a seed point image is input to the region identification unit 58, the region identification unit 58 outputs an image (a region identification image) in which the area of each of the regions is identified. The seed point image is an image in which seed pixels corresponding to each of the regions are identified. In the example of FIG. 6, the seed point image includes seed points attached with label 1 corresponding to the region A, seed points attached with label 2 corresponding to the region B, seed points attached with label 3 corresponding to the region C, seed points attached with label 4 corresponding to the region D, and seed points attached with label 0 for pixels other than the regions A to D. The regions A to D are identified in the region identification image. In the region identification image, each of the regions A to D is one continuous area, and the boundaries between the regions A to D are also clear. A region composed of the regions A to D is a whole region.

The region identification unit 58 is capable of using a segmentation method using cellular automaton based on the seed pixels in the candidate area of each of the identified regions, for example, the GrowCut method, Graph-cuts method, Grab-cut method, or Random Walker method. The GrowCut method is to be described below.

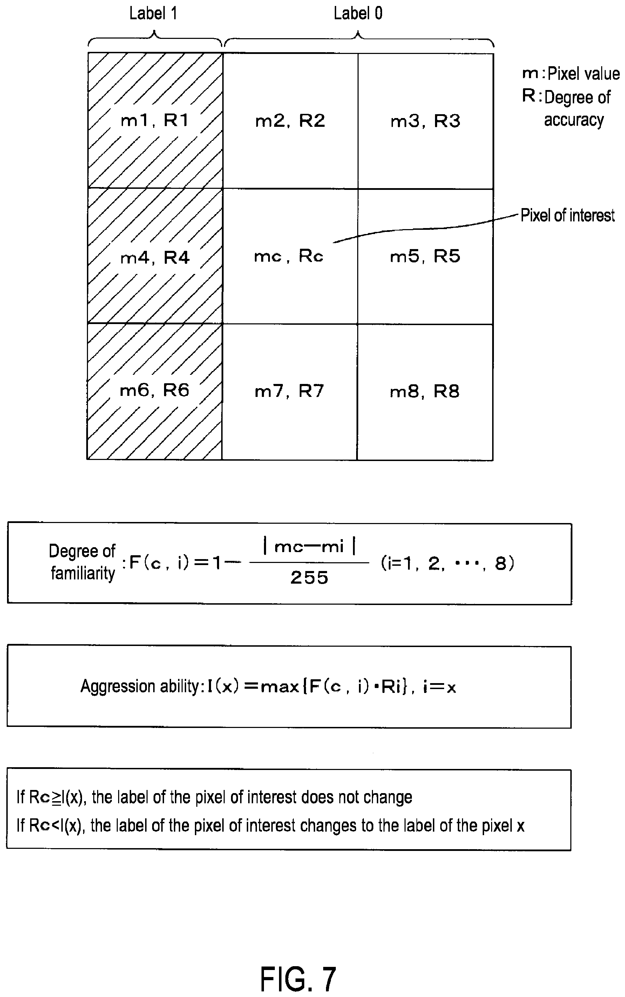

FIG. 7 is a diagram showing an example of the GrowCut method. For convenience, the seed point image is to be described as a 3×3 pixel block composed of a pixel of interest and surrounding eight pixels thereof. mc is regarded as the pixel value of the pixel of interest, and m1 to m8 are regarded as the pixel values of pixels 1 to 8. A pixel value may be a luminance value. In this case, the pixel value may take an integer value in the range of 0 to 255. It is assumed that pixels 1, 4, and 6 are attached with label 1, and the remaining pixels are attached with label 0. Here, the pixels 1, 4, and 6 are assumed to be seed pixels (seed points). Rc and R1 to R8 are regarded as the degree of accuracy (reliability) of the labels of the pixel of interest and pixels 1 to 8, respectively. The degree of accuracy may take real values in the range 0 to 1. The GrowCut method uses the degree of familiarity F with adjacent pixels. F(c, i)=1-|mc-mi|/255 (i=1, 2, ...,8) represents the degree of familiarity of eight pixels adjacent to the pixel of interest. For a pixel x with the largest product of a degree of accuracy Ri and the degree of familiarity F(c, i) of the adjacent pixel, the aggression ability I(x) is represented by I(x)=max{F(c, i) · Ri} and i=x.

The region identification unit 58 changes the label and the accuracy of the pixel according to the magnitude of the degree of accuracy Rc of the pixel of interest and the aggression ability I(x) of the adjacent pixel x. For example, if Rc≧I(x), the label of the pixel of interest (label 0 in this case) and the degree of accuracy Rc do not change. On the other hand, if Rc<I(x), the label of the pixel of interest changes to the label of the pixel x, and the degree of accuracy of the pixel of interest is updated with I(x). If the pixel x is pixel 1, the label of the pixel of interest changes to 1. The region identification unit 58 scans the entire image by moving the pixel of interest and performs similar processing.

As described above, in the GrowCut method, merely by selecting pixels with a high degree of accuracy (for example, 1) as seed pixels (seed points) in the region of the image, segmentation is automatically performed, and each pixel other than the seed pixels may be identified as to which region the pixel is. In the example of FIG. 7, segmentation into two regions with labels 1 and 0 is taken as an example, but segmentation into three or more regions may also be performed in the same manner.

Also, the learning model 61 may be generated as follows. First, medical image data including multiple regions and segmentation image data created by making annotation based on the medical image data are prepared. A segmentation image is an image that indicates to which region each pixel of a medical image belongs, and may be generated by manual input or the like. Seed pixels (seed points) in the segmentation image are selected. The selection of seed pixels may be performed automatically by using the seed pixel selection unit 57, or may be performed manually. When the segmentation image in which the seed pixels are selected is input to the region identification unit 58, the region identification unit 58 outputs an image (a region identification image) in which each of the regions is identified. The medical image data including the regions described above and the area data (teacher data) indicating the area of each of the regions output by the region identification unit 58 are used as training data. A lot of training data are prepared. When the medical image data is input to the learning model 61 based on the training data, the learning model 61 may be generated by updating the internal parameters of the learning model 61 so that the area data of each of the regions output by the learning model 61 approaches the teacher data. In this way, when the medical image data including the regions is input, the learning model 61 may identify each of the regions, and the region identification unit 58 using the GrowCut method or the like may be removed.

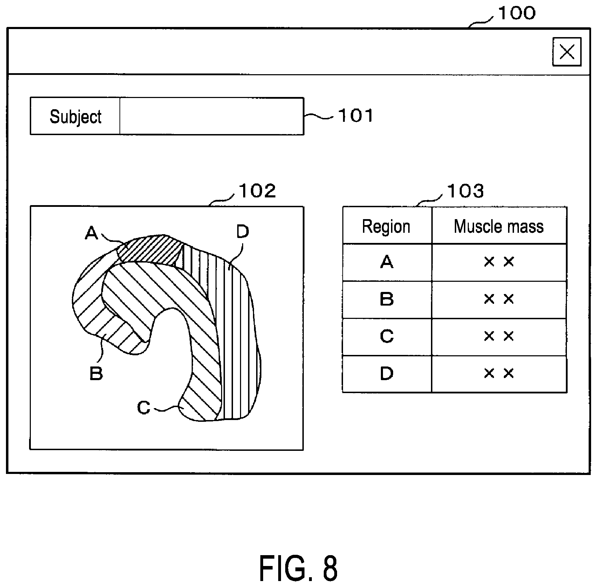

FIG. 8 is a diagram showing an example of a region identification screen 100. On the region identification screen 100 displayed on the display unit 55, a display field 101 for subject information (for example, subject ID, name, etc.), a medical image display field 102 with an identified region, a measurement result field 103, and the like are displayed. In the medical image display field 102, for example, the rectus femoris muscle A, the vastus medialis muscle B, the vastus intermedius muscle C, and the vastus lateralis muscle D of the quadriceps femoris muscle are clearly displayed, and each boundary thereof is also relatively clearly displayed.

The processing unit 51 may display each of the identified regions in a different manner. In the example of FIG. 8, the rectus femoris muscle A, the vastus medialis muscle B, the vastus intermedius muscle C, and the vastus lateralis muscle D are displayed in different patterns for convenience, for example, each of the regions may be displayed in a different color. In this way, each of the regions may be visually recognized easily.

In this way, even if the area for each of the regions cannot be clearly identified merely by the area identification unit 56, among the candidate area of each of the regions identified by the area identification unit 56, for example, by inputting a pixel of which accuracy is equal to or higher than a threshold as a seed pixel (a seed point) to the region identification unit 58, each of the regions may be identified with high accuracy. In particular, when an ultrasound image is used as a medical image, the area of each of the regions is often unclear. However, according to the embodiment, by using the region identification unit 58, the boundary of each of the regions becomes clear, and the area for each of the regions may be identified with high accuracy. In addition, manual setting of seed points may be removed, and the speed of processing may be increased.

The measurement result field 103 may display the muscle mass of each of the rectus femoris muscle A, the vastus medialis muscle B, the vastus intermedius muscle C, and the vastus lateralis muscle D. The muscle mass may be calculated based on the size of each of the regions.

Next, retraining of the learning model 61 is to be described. Training data (second training data) used to generate the learning model 61 by retraining includes medical image data including multiple regions and teacher data corresponding to the medical image data. Area data (segmentation image data) of each of the regions identified by the region identification unit 58 is used as the teacher data. A large amount of the medical image data and the teacher data is prepared to retrain the learning model 61. That is, when the medical image data is input to the learning model 61, the learning model 61 may be retrained (be generated) by updating the internal parameters of the learning model 61 so that the area data of each of the regions output by the learning model 61 approaches the teacher data. In this way, when the medical image data including the regions is input to the learning model 61, the candidate area of each of the regions identified by the learning model 61 has fewer defects and outliers, and is more likely to match the actual area of each of the regions. As a result, the number of seed pixels input to the region identification unit 58 may be increased or the degree of accuracy that the seed pixel is a region of identification may be increased, thereby further improving the accuracy of identifying each of the regions.

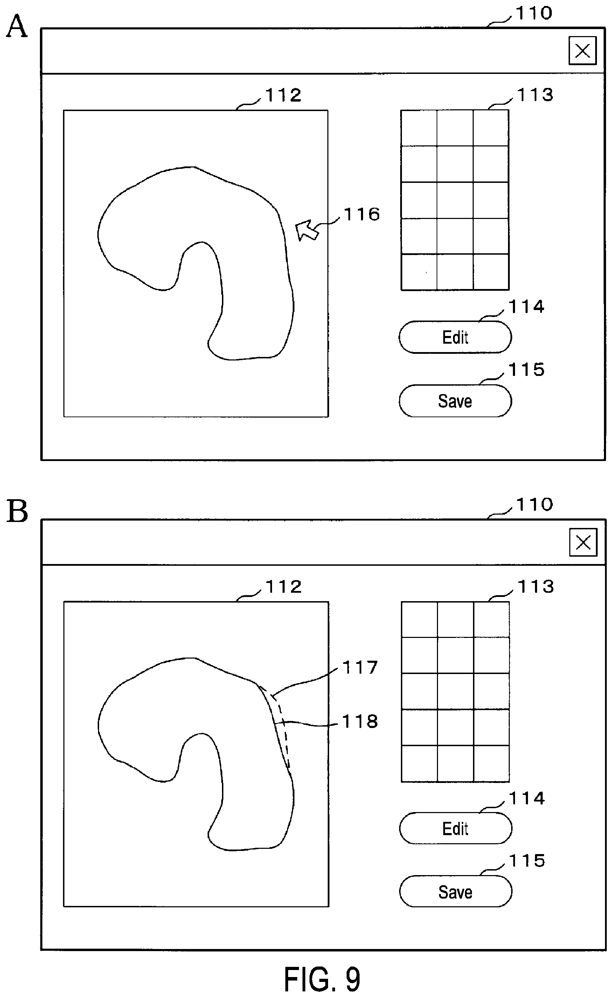

FIG. 9 is a diagram showing an example of an area correction screen 110 of a region. A medical image display field 112, an editing tool 113, an "edit" icon 114, and a "save" icon 115 are displayed on the area correction screen 110. In the medical image display field 112, the region (may be one region or multiple regions) identified by the region identification unit 58 or the candidate area (may be one candidate area or multiple candidate areas) of the region identified by the area identification unit 56 may be displayed. As shown by A of FIG. 9, a user (e.g., a doctor) operates the "edit" icon 114, a cursor 116 is moved to the desired position of the region (or the candidate area of the region), and editing tools in the editing tool 113 are used to correct the area of the region. The editing tools include icons for various tools, for example, a brush for drawing free lines by dragging operation on the image, a shape selection icon for drawing lines, curves, squares, circles, arrows, etc. on the image, a palette icon for changing the size of the pen tip, pattern or color, and an eraser for erasing a desired portion of the image.

For example, as shown by B of FIG. 9, a boundary 117 of the area of the region may be corrected to become a boundary 118. When the correction is completed, the user may save the area data (the segmentation image data) of each of the regions after the correction by operating the "save" icon 115. The storage destination may be the storage unit 60 or an external data server or the like. The area data of each of the regions after the correction is stored in association with the corresponding medical image data, and may be used as training data for retraining of the learning model 61.

That is, when the medical image data is input to the learning model 61, the retraining processing unit 59 may retrain the learning model 61 by updating the internal parameters of the learning model 61 so that the candidate area image data output by the learning model 61 approaches the area data of each of the regions after the correction.

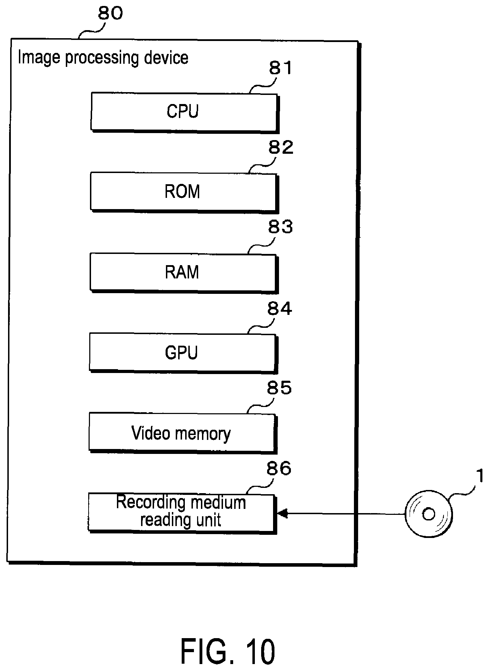

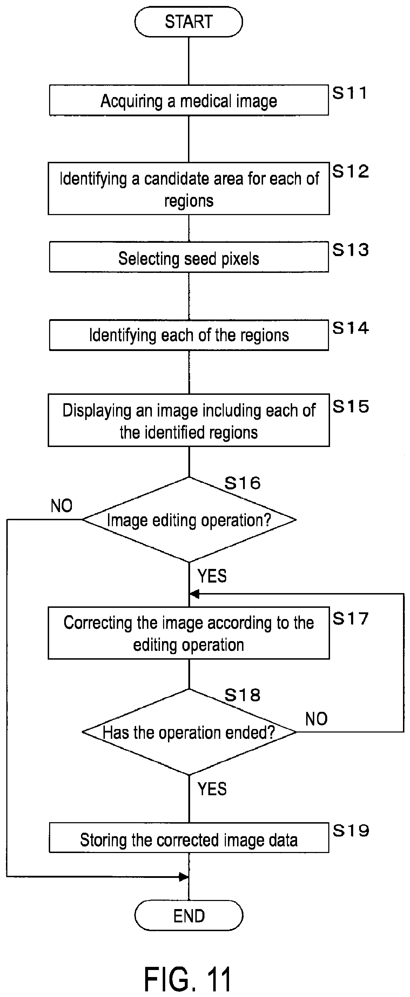

FIG. 10 is a diagram showing another example of the configuration of an image processing device 80 of the embodiment. As shown in FIG. 10, the image processing device 80 may use, for example, a personal computer. The image processing device 80 may be composed of a CPU 81, a ROM 82, a RAM 83, a GPU 84, a video memory 85, a recording medium reading unit 86, and the like. A computer program (a computer program product) recorded on a recording medium 1 (for example, an optically readable disk storage medium such as a CD-ROM) may be read by a recording medium reading unit 86 (for example, an optical disc drive) and stored in the RAM 83. Here, the computer program (the computer program product) includes the processing procedure described in FIG. 11, which is to be described later. The computer program (the computer program product) may be stored in a hard disk (not shown) and stored in the RAM 83 when the program is executed.

By causing the CPU 81 to execute the computer program (the computer program product) stored in the RAM 83, each process in the input unit 52, the operation unit 53, and the processing unit 51 (the area identification unit 56, the region identification unit 58, and the retraining processing unit 59) may be executed. The video memory 85 may temporarily store data and processing results for various image processing. Further, the computer program (the computer program product), instead of being read by the recording medium reading unit 86, may also be downloaded from other computers, network devices, etc. over a network such as the Internet.

FIG. 11 is a flowchart showing a first example of the processing procedure of the image processing device 50. For the sake of convenience, the main body of processing is to be described as the processing unit 51. The processing unit 51 acquires a medical image (S11) and identifies a candidate area for each of regions (S12). The processing unit 51 selects seed pixels from the identified candidate area (S13). The seed pixels may be selected by using the method illustrated in FIG. 4 or FIG. 5.

The processing unit 51 identifies each of the regions based on the selected seed pixels (S14), and displays an image including each of the identified regions (S15). The processing unit 51 determines whether or not image editing operation exists (S16). If the editing operation exists (YES in S16), the image is corrected according to the editing operation (S17), and whether or not the operation has ended is determined (S18). Whether or not the operation has ended may be determined by whether or not the "save" icon 115 has been operated.

If the operation has not ended (NO in S18), the processing unit 51 continues the process of step S17. If the operation is completed (YES in S18), the processing unit 51 stores the corrected image data (S19), and terminates the process. If no editing operation exists (NO in S16), the processing unit 51 terminates the process.

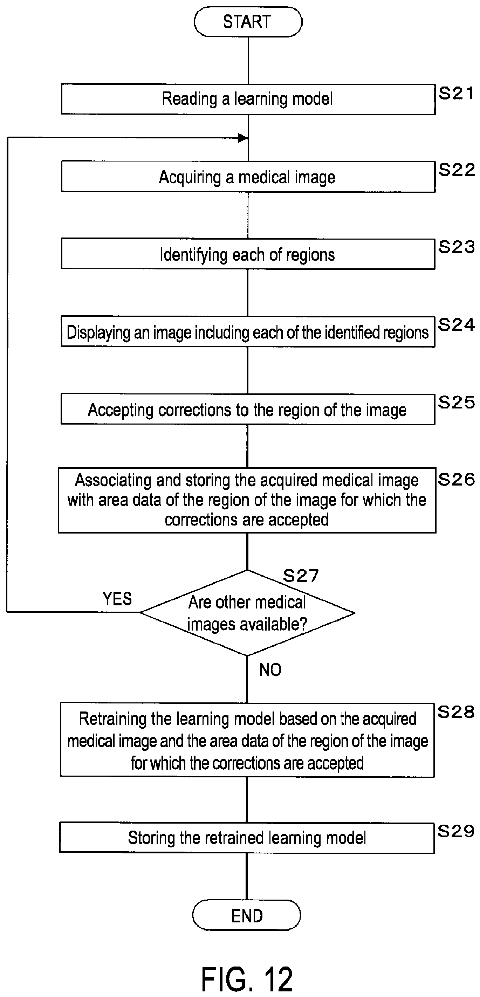

FIG. 12 is a flowchart showing a second example of the processing procedure of the image processing device 50. The process shown in FIG. 12 shows an operational case in which a user such as a doctor uses the image processing device 50 to diagnose a patient. The processing unit 51 reads the learning model 61 from the storage unit 60 (S21), acquires a medical image (S22), and identifies each of regions (S23). Identification of each of the regions is performed by the area identification unit 56 and the region identification unit 58.

The processing unit 51 displays an image including each of the identified regions (S24), and accepts corrections to the region of the image by the user (S25). The number of regions that accept corrections may be one or more. The processing unit 51 associates the acquired medical image with the area data of the region of the image for which the corrections are accepted, and stores the same in the storage unit 60 (S26).

The processing unit 51 determines whether or not there are other medical images available (S27), and if there are other medical image (YES in S27), the process continues from step S22 onward. If there are no other medical images (NO in S27), the processing unit 51 retrains the learning model 61 based on the acquired medical image and the area data of the region of the image for which the corrections are accepted (S28). The processing unit 51 stores the learning model 61 in the storage unit 60 (S29), and terminates the process. Retraining of the learning model 61 may be repeated by repeating the process shown in FIG. 12 as appropriate.

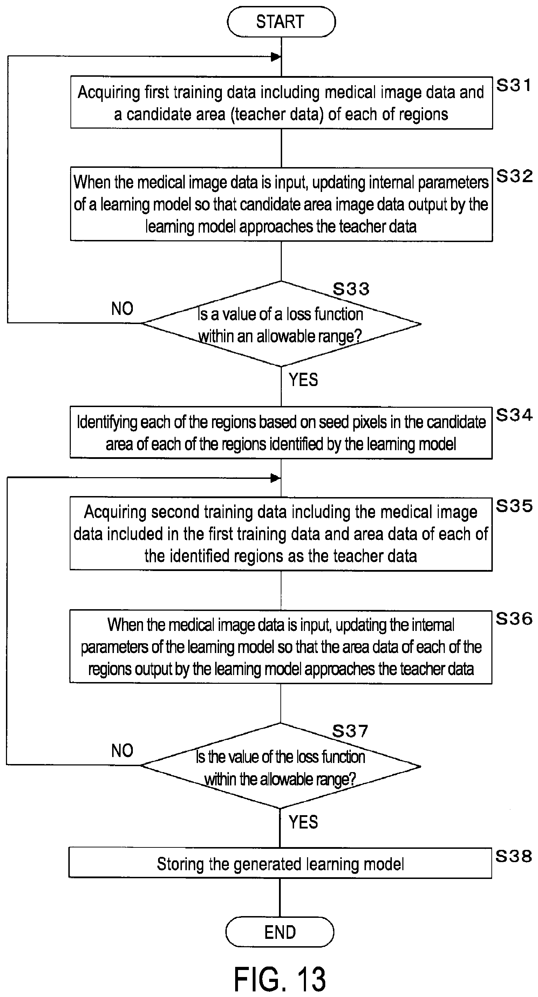

FIG. 13 is a flowchart showing a first example of the processing procedure of the learning model 61 generation method. The learning model 61 may be generated by the image processing device 50 or by another device, but for the sake of convenience, the processing unit 51 is to be described below as the main body of processing. The processing unit 51 acquires first training data including medical image data and a candidate area (teacher data) of each of regions (S31). When the processing unit 51 inputs the medical image data to the learning model 61, internal parameters of the learning model 61 are updated so that the candidate area image data output by the learning model 61 approaches the teacher data (S32). In this case, the internal parameters are adjusted so that a value of a loss function based on the candidate area image data output by the learning model 61 and the teacher data is minimized.

The processing unit 51 determines whether or not the value of the loss function is within an allowable range (S33), and if the value of the loss function is not within the allowable range (NO in S33), the process continues from step S31 onward. If the value of the loss function is within the allowable range (YES in S33), the processing unit 51 identifies each of the regions based on the seed pixels in the candidate area of each of the regions identified by the learning model 61 (S34). Each of the regions may be identified by the region identification unit 58. A large amount of second training data, which is to be described later, may be collected by performing the process from steps S31 to S34.

The processing unit 51 acquires the second training data including the medical image data included in the first training data and the area data of each of the identified regions as the teacher data (S35). When the processing unit 51 inputs the medical image data to the learning model 61, the internal parameters of the learning model 61 are updated so that the area data of each of the regions output by the learning model 61 approaches the teacher data (S36). In this case, the internal parameters are adjusted so that the value of the loss function based on the area data of each of the regions output by the learning model 61 and the teacher data is minimized.

The processing unit 51 determines whether or not the value of the loss function is within the allowable range (S37), and if the value of the loss function is not within the allowable range (NO in S37), the process continues from step S35 onward. If the value of the loss function is within the allowable range (YES in S37), the processing unit 51 stores the generated learning model 61 in the storage unit 60 (S38), and terminates the process.

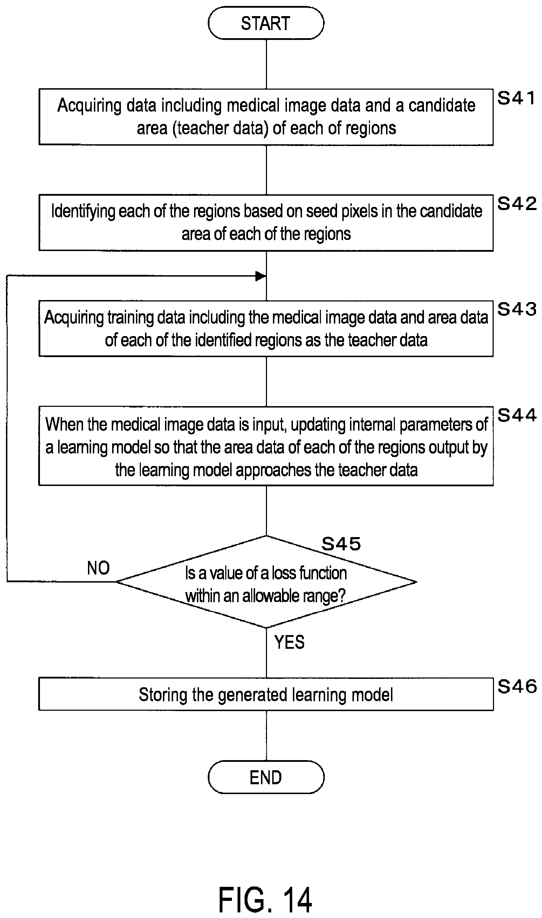

FIG. 14 is a flowchart showing a second example of the processing procedure of the learning model 61 generation method. The processing unit 51 acquires data including medical image data and a candidate area (teacher data) of each of regions (S41). In this case, the data of the candidate area of each of the regions is segmentation image data created by making annotation based on the medical image data. The processing unit 51 identifies each of the regions based on the seed pixels in the candidate area of each of the regions (S42). Each of the regions may be identified by the region identification unit 58.

The processing unit 51 acquires training data including the medical image data and area data of each of the identified regions as the teacher data (S43). When the processing unit 51 inputs the medical image data to the learning model 61, the internal parameters of the learning model 61 are updated so that the area data of each of the regions output by the learning model 61 approaches the teacher data (S44). In this case, the internal parameters are adjusted so that the value of the loss function based on the area data of each of the regions output by the learning model 61 and the teacher data is minimized.

The processing unit 51 determines whether or not the value of the loss function is within an allowable range (S45), and if the value of the loss function is not within the allowable range (NO in S45), the process continues from step S43 onward. If the value of the loss function is within the allowable range (YES in S45), the processing unit 51 stores the generated learning model 61 in the storage unit 60 (S46), and terminates the process.

In the embodiment, a medical image including the quadriceps femoris muscle has been described, but the region is not limited to the quadriceps femoris muscle (thigh), and the imaging target may be the upper arm or the abdomen. Also, a medical image containing cells or the like may be used. Moreover, the image is not limited to a medical image, and may be an image for identifying an object, for example, the embodiment may be applied to an inspection image.

### Reference Signs List

50: image processing device
51: processing unit
52: input unit
53: operation unit
54: image processing unit
55: display unit
56: area identification unit
57: seed pixel selection unit
58: region identification unit
59: retraining processing unit
60: storage unit
61: learning model
601, 602, 603, 604, 605: encoders
606, 607, 608, 609: decoders
80: image processing device
81: CPU
82: ROM
83: RAM
84: GPU
85: video memory
86: recording medium reading unit

## Claims

1. A computer program, causing a computer to execute comprising:
acquiring a medical image comprising a plurality of biological body regions;
individually identifying a candidate area for each of the biological body regions by inputting the medical image to a learning model (61) for identifying the candidate area for each of the biological body regions when the medical image is input;
selecting seed pixels based on a degree of accuracy for pixels in the candidate area for each of the biological body regions which is identified to be in each of the regions,
wherein when a plurality of candidate areas is identified for one of the biological body regions, the seed pixels are selected from a largest candidate area among the plurality of candidate areas; and
individually identifying areas for each of the biological body regions by a GrowCut method using cellular automaton based on the seed pixels selected for each of the biological body regions from the corresponding candidate areas.

2. The computer program according to claim 1, further comprising:
displaying the area for each of the biological body regions which is identified in a different manner, wherein the displaying preferably includes displaying the muscle mass of each of the rectus femoris muscle, the vastus medialis muscle, the vastus intermedius muscle, and the vastus lateralis muscle.

3. The computer program according to claim 1 or 2, further comprising:
retraining the learning model (61) based on the medical image comprising the plurality of biological body regions and area data indicating the area for each of the biological body regions which is identified.

4. The computer program according to any one of claims 1 to 3, further comprising:
accepting a correction to the area for each of the biological body regions which is identified from a user.

5. The computer program according to claim 4, further comprising:
retraining the learning model (61) based on the medical image which is acquired and area data indicating the area for each of the biological body regions for which the correction is accepted.

6. The computer program according to any one of claims 1 to 5, wherein
the medical image comprises an ultrasound image.

7. The computer program according to any one of claims 1 to 6, wherein
an imaging target of the medical image is any of a thigh, an upper arm, and an abdomen.

8. A learning model generation method, comprising:
acquiring first training data comprising a medical image comprising a plurality of biological body regions and a candidate area for each of the biological body regions (S31);
selecting seed pixels based on a degree of accuracy for pixels in the candidate area for each of the biological body regions which is identified to be in each of the regions,
wherein when a plurality of candidate areas is identified for one of the biological body regions, the seed pixels are selected from a largest candidate area among the plurality of candidate areas;
generating a learning model (61) to identify the candidate area for each of the biological body regions when the medical image comprising the plurality of regions is input, based on the first training data;
individually identifying an area for each of the biological body regions by a GrowCut method using cellular automaton based on the seed pixels selected for each of the biological body regions which is identified by the learning model (61) (S34) from the corresponding candidate areas; and
generating the learning model (61) to identify the area for each of the biological body regions when the medical image comprising the plurality of biological body regions is input, based on second training data comprising the medical image comprising the plurality of biological body regions and area data indicating the area for each of the biological body regions which is identified.

9. An image processing method, comprising:
acquiring a medical image comprising a plurality of biological body regions (S11);
identifying a candidate area for each of the biological body regions by inputting the medical image to a learning model (61) for identifying the candidate area for each of the biological body regions when the medical image is input (S12);
selecting seed pixels based on a degree of accuracy for pixels in the candidate area for each of the biological body regions which is identified to be in each of the regions,
wherein when a plurality of candidate areas is identified for one of the biological body regions, the seed pixels are selected from a largest candidate area among the plurality of candidate areas;
and
individually identifying an area for each of the biological body regions by a GrowCut method using cellular automaton based on the seed pixels selected for each of the biological body regions (S14) from the corresponding candidate areas.

10. An image processing device (50, 80), comprising:
an acquisition unit configured to acquire a medical image comprising a plurality of biological body regions;
a unit for selecting seed pixels based on a degree of accuracy for pixels in the candidate area for each of the biological body regions which is identified to be in each of the regions,
wherein when a plurality of candidate areas is identified for one of the biological body regions, the seed pixels are selected from a largest candidate area among the plurality of candidate areas;
an area identification unit (56) configured to identify a candidate area for each of the biological body regions by inputting the medical image to a learning model (61) for identifying the candidate area for each of the biological body regions when the medical image is input; and
a region identification unit (58) configured to individually identify an area for each of the biological body regions by a GrowCut method using cellular automaton based on the seed pixels selected for each of the biological body regions from the corresponding candidate areas.

## Patentansprüche

1. Computerprogramm, das veranlasst, dass ein Computer Folgendes ausführt, aufweisend:
Erfassen eines medizinischen Bildes, das eine Vielzahl von biologischen Körperregionen aufweist;
individuelles Identifizieren eines Kandidatenbereichs für jede der biologischen Körperregionen durch Eingeben des medizinischen Bildes in ein Lernmodell (61) zum Identifizieren des Kandidatenbereichs für jede der biologischen Körperregionen, wenn das medizinische Bild eingegeben wird;
Auswählen von Seed-Pixeln basierend auf einem Genauigkeitsgrad für Pixel in dem Kandidatenbereich für jede der biologischen Körperregionen, der als in jeder der Regionen befindlich identifiziert wird,
wobei, wenn eine Vielzahl von Kandidatenbereichen für eine der biologischen Körperregionen identifiziert wird, die Seed-Pixel aus einem größten Kandidatenbereich unter der Vielzahl von Kandidatenbereichen ausgewählt werden; und
individuelles Identifizieren von Bereichen für jede der biologischen Körperregionen durch ein GrowCut-Verfahren unter Verwendung eines zellularen Automaten basierend auf den Seed-Pixeln, die für jede der biologischen Körperregionen aus den entsprechenden Kandidatenbereichen ausgewählt werden.

2. Computerprogramm nach Anspruch 1, ferner aufweisend:
Anzeigen des Bereichs für jede der biologischen Körperregionen, der auf eine andere Weise identifiziert wird, wobei das Anzeigen vorzugsweise das Anzeigen der Muskelmasse von jedem von dem Rectus femoris Muskel, dem Vastus medialis Muskel, dem Vastus intermedius Muskel und dem Vastus lateralis Muskel umfasst.

3. Computerprogramm nach Anspruch 1 oder 2, ferner aufweisend:
Neutrainieren des Lernmodells (61) basierend auf dem medizinischen Bild, das die Vielzahl von biologischen Körperregionen und Bereichsdaten aufweist, die den Bereich für jede der biologischen Körperregionen angeben, der identifiziert wird.

4. Computerprogramm nach einem der Ansprüche 1 bis 3, ferner aufweisend:
Akzeptieren einer Korrektur des Bereichs für jede der biologischen Körperregionen, der von einem Benutzer identifiziert wird.

5. Computerprogramm nach Anspruch 4, ferner aufweisend:
Neutrainieren des Lernmodells (61) basierend auf dem medizinischen Bild, das erfasst wird, und Bereichsdaten, die den Bereich für jede der biologischen Körperregionen angeben, für die die Korrektur akzeptiert wird.

6. Computerprogramm nach einem der Ansprüche 1 bis 5, wobei
das medizinische Bild ein Ultraschallbild aufweist.

7. Computerprogramm nach einem der Ansprüche 1 bis 6, wobei
ein Bildgebungsziel des medizinischen Bildes ein beliebiges von einem Oberschenkel, einem Oberarm und einem Abdomen ist.

8. Lernmodell-Erzeugungsverfahren, aufweisend:
Erfassen erster Trainingsdaten, die ein medizinisches Bild aufweisen, das eine Vielzahl von biologischen Körperregionen und einen Kandidatenbereich für jede der biologischen Körperregionen aufweist (S31);
Auswählen von Seed-Pixeln basierend auf einem Genauigkeitsgrad für Pixel in dem Kandidatenbereich für jede der biologischen Körperregionen, der als in jeder der Regionen befindlich identifiziert wird,
wobei, wenn eine Vielzahl von Kandidatenbereichen für eine der biologischen Körperregionen identifiziert wird, die Seed-Pixel aus einem größten Kandidatenbereich unter der Vielzahl von Kandidatenbereichen ausgewählt werden;
Erzeugen eines Lernmodells (61) zum Identifizieren des Kandidatenbereichs für jede der biologischen Körperregionen, wenn das medizinische Bild, das die Vielzahl von Regionen aufweist, eingegeben wird, basierend auf den ersten Trainingsdaten;
individuelles Identifizieren eines Bereichs für jede der biologischen Körperregionen durch ein GrowCut-Verfahren unter Verwendung eines zellularen Automaten basierend auf den Seed-Pixeln, die für jede der biologischen Körperregionen ausgewählt werden, die durch das Lernmodell (61) identifiziert wird (S34), aus den entsprechenden Kandidatenbereichen; und
Erzeugen des Lernmodells (61) zum Identifizieren des Bereichs für jede der biologischen Körperregionen, wenn das medizinische Bild, das die Vielzahl von biologischen Körperregionen aufweist, eingegeben wird, basierend auf zweiten Trainingsdaten, die das medizinische Bild aufweisen, das die Vielzahl von biologischen Körperregionen und Bereichsdaten aufweist, die den Bereich für jede der biologischen Körperregionen angeben, der identifiziert wird.

9. Bildverarbeitungsverfahren, aufweisend:
Erfassen eines medizinischen Bildes, das eine Vielzahl von biologischen Körperregionen aufweist (S11);
Identifizieren eines Kandidatenbereichs für jede der biologischen Körperregionen durch Eingeben des medizinischen Bildes in ein Lernmodell (61) zum Identifizieren des Kandidatenbereichs für jede der biologischen Körperregionen, wenn das medizinische Bild eingegeben wird (S12);
Auswählen von Seed-Pixeln basierend auf einem Genauigkeitsgrad für Pixel in dem Kandidatenbereich für jede der biologischen Körperregionen, der als in jeder der Regionen befindlich identifiziert wird,
wobei, wenn eine Vielzahl von Kandidatenbereichen für eine der biologischen Körperregionen identifiziert wird, die Seed-Pixel aus einem größten Kandidatenbereich unter der Vielzahl von Kandidatenbereichen ausgewählt werden;
und
individuelles Identifizieren eines Bereichs für jede der biologischen Körperregionen durch ein GrowCut-Verfahren unter Verwendung eines zellularen Automaten basierend auf den Seed-Pixeln, die für jede der biologischen Körperregionen ausgewählt werden (S14), aus den entsprechenden Kandidatenbereichen.

10. Bildverarbeitungsvorrichtung (50, 80), aufweisend:
eine Erfassungseinheit, die konfiguriert ist, um ein medizinisches Bild zu erfassen, das eine Vielzahl von biologischen Körperregionen aufweist;
eine Einheit zum Auswählen von Seed-Pixeln basierend auf einem Genauigkeitsgrad für Pixel in dem Kandidatenbereich für jede der biologischen Körperregionen, der als in jeder der Regionen befindlich identifiziert wird,
wobei, wenn eine Vielzahl von Kandidatenbereichen für eine der biologischen Körperregionen identifiziert wird, die Seed-Pixel aus einem größten Kandidatenbereich unter der Vielzahl von Kandidatenbereichen ausgewählt werden;
eine Bereichsidentifikationseinheit (56), die konfiguriert ist, um einen Kandidatenbereich für jede der biologischen Körperregionen durch Eingeben des medizinischen Bildes in ein Lernmodell (61) zum Identifizieren des Kandidatenbereichs für jede der biologischen Körperregionen zu identifizieren, wenn das medizinische Bild eingegeben wird; und
eine Regionsidentifikationseinheit (58), die konfiguriert ist, um einen Bereich für jede der biologischen Körperregionen durch ein GrowCut-Verfahren unter Verwendung eines zellularen Automaten basierend auf den Seed-Pixeln, die für jede der biologischen Körperregionen aus den entsprechenden Kandidatenbereichen ausgewählt werden, individuell zu identifizieren.

## Revendications

1. Programme informatique amenant un ordinateur à exécuter un traitement comprenant :
l'acquisition d'une image médicale comprenant une pluralité de régions biologiques du corps ;
l'identification individuelle d'une zone candidate pour chacune des régions biologiques du corps en entrant l'image médicale dans un modèle d'apprentissage (61) afin d'identifier la zone candidate pour chacune des régions biologiques du corps lorsque l'image médicale est entrée ;
la sélection de pixels de départ sur la base d'un degré de précision pour des pixels dans la zone candidate pour chacune des régions biologiques du corps qui est identifiée comme étant dans chacune des régions,
dans lequel, lorsqu'une pluralité de zones candidates est identifiée pour l'une des régions biologiques du corps, les pixels de départ sont sélectionnés dans une zone candidate la plus grande parmi la pluralité de zones candidates ; et
l'identification individuelle de zones pour chacune des régions biologiques du corps par un procédé GrowCut utilisant un automate cellulaire basé sur les pixels de départ sélectionnés pour chacune des régions biologiques du corps dans les zones candidates correspondantes.

2. Programme informatique selon la revendication 1, comprenant en outre :
l'affichage de la zone pour chacune des régions biologiques du corps qui est identifiée de manière différente, dans lequel l'affichage comporte de préférence l'affichage de la masse musculaire de chacun du muscle droit fémoral, du muscle vaste interne, du muscle crural et du muscle vaste externe.

3. Programme informatique selon la revendication 1 ou 2, comprenant en outre :
le réentraînement du modèle d'apprentissage (61) sur la base de l'image médicale comprenant la pluralité de régions biologiques du corps et de données de zone indiquant la zone pour chacune des régions biologiques du corps qui est identifiée.

4. Programme informatique selon l'une des revendications 1 à 3, comprenant en outre :
l'acceptation, de la part d'un utilisateur, d'une correction de la zone pour chacune des régions biologiques du corps qui est identifiée.

5. Programme informatique selon la revendication 4, comprenant en outre :
le réentraînement du modèle d'apprentissage (61) sur la base de l'image médicale acquise et de données de zone indiquant la zone pour chacune des régions biologiques du corps pour lesquelles la correction est acceptée.

6. Programme informatique selon l'une des revendications 1 à 5, dans lequel
l'image médicale comprend une image échographique.

7. Programme informatique selon l'une des revendications 1 à 6, dans lequel
une cible d'imagerie de l'image médicale est l'un quelconque parmi une cuisse, le haut d'un bras et un abdomen.

8. Procédé de génération de modèle d'apprentissage, comprenant :
l'acquisition de premières données d'entraînement comprenant une image médicale comprenant une pluralité de régions biologiques du corps et une zone candidate pour chacune des régions biologiques du corps (S31) ;
la sélection de pixels de départ sur la base d'un degré de précision pour des pixels dans la zone candidate pour chacune des régions biologiques du corps qui est identifiée comme étant dans chacune des régions,
dans lequel, lorsqu'une pluralité de zones candidates est identifiée pour l'une des régions biologiques du corps, les pixels de départ sont sélectionnés dans une zone candidate la plus grande parmi la pluralité de zones candidates ;
la génération d'un modèle d'apprentissage (61) pour identifier la zone candidate pour chacune des régions biologiques du corps lorsque l'image médicale comprenant la pluralité de régions est entrée, sur la base des premières données d'entraînement ;
l'identification individuelle d'une zone pour chacune des régions biologiques du corps par un procédé GrowCut utilisant un automate cellulaire basé sur les pixels de départ sélectionnés pour chacune des régions biologiques du corps qui est identifiée par le modèle d'apprentissage (61) (S34) dans les zones candidates correspondantes ; et
la génération du modèle d'apprentissage (61) pour identifier la zone pour chacune des régions biologiques du corps lorsque l'image médicale comprenant la pluralité de régions biologiques du corps est entrée, sur la base de deuxièmes données d'entraînement comprenant l'image médicale comprenant la pluralité de régions biologiques du corps et des données de zone indiquant la zone pour chacune des régions biologiques du corps qui est identifiée.

9. Procédé de traitement d'image, comprenant :
l'acquisition d'une image médicale comprenant une pluralité de régions biologiques du corps (S11) ;
l'identification d'une zone candidate pour chacune des régions biologiques du corps en entrant l'image médicale dans un modèle d'apprentissage (61) afin d'identifier la zone candidate pour chacune des régions biologiques du corps lorsque l'image médicale est entrée (S12) ;
la sélection de pixels de départ sur la base d'un degré de précision pour des pixels dans la zone candidate pour chacune des régions biologiques du corps qui est identifiée comme étant dans chacune des régions,
dans lequel, lorsqu'une pluralité de zones candidates est identifiée pour l'une des régions biologiques du corps, les pixels de départ sont sélectionnés dans une zone candidate la plus grande parmi la pluralité de zones candidates ; et
l'identification individuelle d'une zone pour chacune des régions biologiques du corps par un procédé GrowCut utilisant un automate cellulaire basé sur les pixels de départ sélectionnés pour chacune des régions biologiques du corps (S14) dans les zones candidates correspondantes.

10. Dispositif de traitement d'image (50, 80), comprenant :
une unité d'acquisition configurée pour acquérir une image médicale comprenant une pluralité de régions biologiques du corps ;
une unité pour sélectionner des pixels de départ sur la base d'un degré de précision pour des pixels dans la zone candidate pour chacune des régions biologiques du corps qui est identifiée comme étant dans chacune des régions,
dans lequel, lorsqu'une pluralité de zones candidates est identifiée pour l'une des régions biologiques du corps, les pixels de départ sont sélectionnés dans une zone candidate la plus grande parmi la pluralité de zones candidates ;
une unité d'identification de zone (56) configurée pour identifier une zone candidate pour chacune des régions biologiques du corps en entrant l'image médicale dans un modèle d'apprentissage (61) afin d'identifier la zone candidate pour chacune des régions biologiques du corps lorsque l'image médicale est entrée ; et
une unité d'identification de région (58) configurée pour identifier individuellement une zone pour chacune des régions biologiques du corps par un procédé GrowCut utilisant un automate cellulaire basé sur les pixels de départ sélectionnés pour chacune des régions biologiques du corps dans les zones candidates correspondantes.
